# EUROPÄISCHE PATENTANMELDUNG

(11) **EP 4 541 273 A1**
(43) Veröffentlichungstag der Anmeldung: **23.04.2025**
(21) Anmeldenummer: 23204781.1
(22) Anmeldetag: 20.10.2023
(51) Int. Cl.: A61B 5/083, A61B 5/00

(54) **VERFAHREN BETREFFEND PULMONALE HYPERTONIE**

(71) Anmelder: Justus-Liebig-Universität Gießen, 35390 Gießen (DE); Fuchs, Steffen, 35633 Lahnau (DE); Zorn, Alexander, 35452 Heuchelheim (DE)
(72) Erfinder: Fuchs, Steffen, 35633 Lahnau (DE); Zorn, Alexander, 35452 Heuchelheim (DE); Gall, Henning, 35452 Heuchelheim (DE); Fuchs, Steffen, 35633 Lahnau (DE); Ghofrani, Ardeschir, 35435 Wettenberg (DE); Richter, Manuel, 35398 Gießen (DE); Tello, Khodr, 35423 Lich (DE); Seeger, Werner, 35444 Biebertal (DE); Zorn, Alexander, 35452 Heuchelheim (DE); Yogeswaran, Athiththan, 35392 Gießen (DE)
(74) Vertreter: Stumpf, Peter

(57) **Zusammenfassung**

Verfahren zur Ermittlung eines Scorewertes für die Wahrscheinlichkeit des Vorliegens einer Pulmonalen Hypertonie einer Person basierend auf CO₂-Messdaten über den endtidalen CO₂-Partialdruck über einen Zeitraum und EKG-Messdaten und davon bestimmten Werten des Sokolow-Lyon Index, der S-Zacke in V5, der R-Zacke in V4 und es Winkels der QRS-Achse.

## Beschreibung

Die Erfindung betrifft ein Verfahren zur Bestimmung eines Scorewertes für die Wahrscheinlichkeit des Vorliegens einer Pulmonalen Hypertonie (PH) mit Hilfe des endtidalen Partialdruckes von Kohlenstoffdioxid (PₑₜCO₂).

### Beschreibung und Einleitung des allgemeinen Gebietes der Erfindung

Durch den Lungenkreislauf fließt jederzeit die gleiche Menge Blut wie durch alle restlichen Organe des Körpers zusammen, nämlich circa fünf Liter pro Minute. In Ruhe sind dies circa 7200 Liter pro Tag. Unter Belastung kann diese Blutmenge auf ein Vielfaches gesteigert werden.

Die gesunde Lunge ist wie ein elastischer Schwamm aufgebaut, so dass der Strömungswiderstand in den Lungengefäßen normalerweise sehr gering ist: Um 5 L/min Herzzeitvolumen durch die Lunge zu pumpen, braucht es nur 14mmHg (1866,51 Pa) Druck - um die gleiche Menge durch einen Strohhalm zu pumpen, bräuchte es den etwa 5 fachen Druck. Im direkten Vergleich zum Hochdrucksystem des menschlichen Körpers muss aufgrund des geringen Lungenwiderstandes vom rechten Herzen normalerweise nur ein Sechstel des Systemdrucks aufgebaut werden, um das Blut durch die Lunge zu pumpen.

Für einen effizienten Gasaustausch und somit die Versorgung aller Körperzellen mit Sauerstoff, müssen Lunge und Herz exakt zusammenarbeiten. Störungen in dieser Zusammenarbeit im kardio-pulmonalen System können zur Pulmonalen Hypertonie (PH) führen. Die PH ist somit ein pathophysiologisches Phänomen, das klinisch zu Symptomen wie Dyspnoe (Atemnot) führt. Die Erfahrung im klinischen Alltag zeigt, dass die Einteilung der verschiedenen Formen der PH selbst spezialisierten Ärzten nicht immer eindeutig möglich ist.

Angesichts der steigenden Zahl von Patienten, die Symptome wie z.B. Dyspnoe zeigen und deshalb auf Pulmonale Hypertonie (PH) untersucht werden, und der erheblichen Kosten, des Zeitaufwands und des potenziellen Risikos bei der Beurteilung von Lungengefäßerkrankungen, besteht ein Interesse an der Entwicklung neuer, nicht-invasiver Diagnoseverfahren, um bei Patienten ohne die Risiken durch eine invasive Diagnostik eine Pulmonale Hypertonie (PH) zu identifizieren.

### Stand der Technik

Derzeit erfordert die endgültige Bestätigung der Diagnose PH einen Rechtsherzkatheter (RHK). Obwohl es klinische und echokardiographische Merkmale gibt, die eine PH wahrscheinlicher machen, sind diese Indikatoren oft nicht ausreichend, um auf eine RHK bei Patienten mit erhöhtem rechtsventrikulärem systolischem Druck (RVSD) in der Echokardiographie zu verzichten. Auch ist ein normaler RVSD in der Echokardiographie nicht ausreichend, um eine PH auszuschließen. Die Diagnose der Pulmonalen Hypertonie wird daher leider sehr oft erst zu spät oder gar nicht gestellt, mit weitreichenden Folgen für den individuellen Patienten. Das derzeitige Standardverfahren ist es, zur Diagnostik einer PH zunächst eine Echokardiographie durchzuführen. Diese ist jedoch, wie Vergleichsstudien zeigen trotz des Aufwandes, der beschränkten Verfügbarkeit und der erforderlichen Expertise in Hinsicht auf die Sensitivität, Spezifität und somit Genauigkeit nicht immer ausreichend zuverlässig.

### Aufgabe

Aufgabe der vorliegenden Erfindung ist, die Nachteile des Standes der Technik zu vermindern, so dass die Messung des endtidalen CO₂ Partialdruckes zur Diagnostik für Pulmonale Hypertonie genutzt werden kann. Damit wird eine präzisere und zuverlässigere Diagnose ermöglicht, die nicht-invasiv und kostengünstig für Patienten durchführbar ist.

### Lösung der Aufgabe

Die Lösung dieser Aufgabe ergibt sich aus den Merkmalen des Hauptanspruches. Weiterhin sind vorteilhafte Ausgestaltungen und Weiterbildungen der Erfindung den Unteransprüchen entnehmbar.

Zunächst erfolgt die Definition der für das erfindungsgemäße Verfahren maßgeblichen Begriffe:

### Messdaten über den endtidalen CO₂ Partialdruck

Der Wert des endtidalen CO₂ Partialdruckes (EtCO2), ist ein wichtiger Kennwert, um die Lungenfunktion einschätzen zu können.

Insbesondere ist ein besonders niedriger Wert des endtidalen CO₂ Partialdruckes (EtCO2) ein starker Hinweis, dass eine Pulmonale Hypertonie (PH) vorliegen könnte.

Gemessen wird bei der Kapnometrie der prozentuale Anteil oder der Partialdruck des Kohlendioxids. Der Normbereich für den endexspiratorischen Kohlenstoffdioxid-Partialdruck liegt bei 33 bis 43 mmHg bzw., wenn der Messwert als Konzentration angegeben wird, bei 4,3 bis 5,7 Vol.-%. Die Umrechnung zwischen beiden Einheiten hängt vom Umgebungsluftdruck ab, den das Gerät daher ebenfalls misst. Der Goldstandard zur Messung des CO₂-Partialdrucks im Blut ist die arterielle Blutgasanalyse. Aufgrund ihrer Invasivität erlaubt sie meist nur eine Momentaufnahme und ist daher besonders für außerhalb einer Klinik nur eingeschränkt nutzbar. Zur kontinuierlichen Messung des CO₂ Partialdruckes im Blut kann man jedoch den CO₂ Partialdruck in der Ausatemluft des Patienten messen. Dies wird als Kapnometrie bezeichnet. Die Bestimmung des CO₂ Partialdruckes erfolgt am Ende der maximalen Ausatmung, so dass man von dem endtidalen CO₂-Partialdruck spricht (PₑₜCO₂). Eine weitere, weniger verbreitete Methode, ist die Messung des CO₂ - Wertes über die Haut, die transkutane Kapnometrie (P_{tc}CO₂). Diese Methode der transkutanen Messung des CO₂ bildet nicht die CO₂ der Ausatemluft ab. Sie ist somit nicht für die Untersuchung von Lungenerkrankungen geeignet.

In ihrer Funktion und ihren Ergebnissen bezogen auf den tatsächlichen paCO₂ unterscheiden sich die verschiedenen Verfahren.

Die endtidale Kapnographie ist die am weitesten verbreitete Methode zur nicht - invasiven Messung des CO₂- Partialdruckes. Ihre Hauptanwendungen liegen meist im Rahmen der Überwachung einer Anästhesie oder zur Diagnostik von pulmonalvaskulären Erkrankungen.

Die Messung kann im Hauptstromverfahren (Mainstream) oder im Nebenstromverfahren (Sidestream) des Ventilationskreises mittels Infrarot-Messung erfolgen. Im Mainstream Verfahren wird der CO₂ Spiegel der Ausatemluft des Patienten gemessen. Im Nebenstromverfahren wird durch eine kleine Zusatzleitung eine bestimmte Gasmenge kontinuierlich aus dem Hauptstrom abgezogen und im Analyser des Beatmungsgerätes ausgewertet. Moderne Kapnometer verwenden dafür kleinstmögliche Gasmengen, diese liegen meist zwischen 30-50ml/min.

### Dynamischer klinischer Parameter P1:

Dynamische klinische Parameter P1 können entweder EKG-Messdaten oder eine Kombination von EKG-Messdaten und MRT-Messdaten umfassen.

EKG steht für Elektrokardiographie und bezeichnet eine Untersuchungsmethode, bei der die elektrische Aktivität des Herzens mittels Elektroden auf der Körperoberfläche gemessen wird. Regelmäßige elektrische Impulse steuern den Herzschlag. Sie werden vom Sinusknoten im rechten Vorhof des Herzens ausgelöst und breiten sich wie kleine Stromstöße über den Herzmuskel aus. Dadurch ziehen sich zuerst die Vorhöfe und dann die Herzkammern zusammen. Die Ausbreitung der elektrischen Reize im Herzmuskel ist auch auf der Haut messbar. Ein EKG misst diese elektrischen Potentialunterschiede an verschiedenen Stellen des Körpers und stellt diese als Kurven dar. Diese EKG-Kurven werden Elektrokardiogramm genannt. Wenn das Herz gleichmäßig schlägt, ergibt sich das typische EKG-Muster: Der erste Ausschlag (p-Welle) zeigt, wie sich der elektrische Impuls (Erregung) über die Herzvorhöfe ausbreitet. Die Vorhöfe ziehen sich zusammen, leiten Blut in die Herzkammern und entspannen sich sofort wieder. Die Erregung erreicht dann die Herzkammern. Im EKG ist das als Q-, R- und S-Zacken sichtbar, dem sogenannten QRS-Komplex, der mit der Kontraktion der Herzkammern assoziiert ist. Danach zeigt die T-Welle an, dass sich die Erregung zurückbildet und sich die Herzkammern wieder entspannen.

Die zeitlich eingeordneten Daten des Erregungsablaufes des Herzes werden im Folgenden vereinfacht als EKG-(Mess-)daten bezeichnet.

Das erfindungsgemäße Verfahren zur Bestimmung eines Scorewertes E für die Wahrscheinlichkeit des Vorliegens einer Pulmonalen Hypertonie einer Person P aus dem endtidalen CO₂ Partialdruck umfasst dabei wenigstens die folgenden Schritte
I. Bereitstellen der CO₂-Messdaten über den endtidalen CO₂ Partialdruckes (PetCO₂) der Person (P) über einen Zeitraum (t)
   In einem ersten Schritt werden CO₂ Messdaten D1 über den endtidalen CO₂ Partialdruck bereitgestellt. Die Messung des endtidalen CO₂-Partialdruckes erfolgt dabei aus der ausgeatmeten Atemluft außerhalb des menschlichen Körpers.
   Die Messung kann dabei beispielsweise über eine endtidale Kapnographie erfolgen.
II. Bereitstellen von EKG-Messdaten als dynamischer-klinischer Parameter (P1) und Bestimmung wenigstens des Wertes des Sokolow-Lyon Index , Wertes der S-Zacke in V5, des Wertes der R-Zacke in V4 und Wertes des Winkels der QRS-Achse aus den EKG-Messdaten der Person (P) über den gleichen Zeitraum (t) wie in Schritt I
III. Diese EKG-Messdaten sind dabei die Daten, welche nach Abschluss der EKG Messung an einer Person vorliegen. Das Bereitstellen beginnt erst nach Abschluss der Untersuchung mit Datenerhebung am menschlichen Körper, d.h. erst nachdem die Messung am Patienten abgeschlossen ist. Bestimmung eines Scorewertes (S) für die Wahrscheinlichkeit des Vorliegens einer Pulmonalen Hypertonie dieser Person aus den CO₂-Messdaten über den endtidalen CO₂ Partialdruckes (PₑₜCO₂) und wenigstens der EKG-Messdaten als dynamischer-klinischer Parameter (P1) in folgenden Teilschritten
   a. Vergleich des PetCO₂ aus Schritt I mit einem Grenzwert G1,
      - bei PetCO₂ größer oder gleich G1 ist S gleich 0,
      - bei PetCO₂ kleiner G1, erfolgt eine Weiterführung des Verfahrens in Schritt IIIb,
   b. Vergleich des Wertes Sokolow-Lyon Index aus Schritt II mit einem Grenzwert G2,
      - bei Sokolow-Lyon Index größer oder gleich G2 ist S gleich 1,
      - bei Sokolow-Lyon Index < G1 erfolgt eine Weiterführung des Verfahrens in Schritt IIIc,
   c. Vergleich des Wertes der S-Zacke in V5 aus Schritt II mit einem Grenzwert G3,
      - bei S-Zacke in V5 größer oder gleich G3 ist S gleich 0,
      - bei S-Zacke in V5 kleiner G3 erfolgt eine Weiterführung des Verfahrens in Schritt IIId,
   d. Vergleich des Wertes der R-Zacke in V4 aus Schritt II mit einem Grenzwert G4,
      - bei R-Zacke in V2 kleiner G4, ist S gleich 1,
      - bei R-Zacke in V2 größer oder gleich G4 erfolgt eine Weiterführung des Verfahrens in Schritt IIIe,
   e. Vergleich des Wertes des Winkels der QRS-Achse aus Schritt II mit einem Grenzwert G5,
      - bei Winkel der QRS Achse größer G5 ist S gleich1,
      - bei Winkel der QRS Achse kleiner oder gleich G5 ist S gleich 0.

Die Durchführung des Verfahrens erfolgt durch eine Vorrichtung zur elektronischen Datenverarbeitung PC, Smartphone, Mac^{©}. Es handelt sich um ein computerimplementiertes Verfahren.

Neben klinischen sowie EKG Messdaten wird insbesondere der in den aktuellen Leitlinien empfohlene Screening-Score zur Beurteilung der Wahrscheinlichkeit des Vorliegens einer Pulmonalen Hypertonie bestimmt. In Kombination mit dem endtidalen CO₂ Partialdruck (PₑₜCO₂) kann eine Verbesserung der Vorhersagekraft jedes einzelnen Testes erreicht werden.

Die Bestimmung des Scorewertes erfolgt dabei iterativ.

Zunächst erfolgt eine Klassifikation anhand des PetCO₂.

Ein Entscheidungsbaum zur Klassifizierung in die Gruppen "PH wahrscheinlich" und "PH unwahrscheinlich" erfolgt zunächst anhand des Messdaten des endtidalen CO2-Wertes (PetCO₂). Der in der Fachwelt anerkannter Grenzwert G1 liegt bei etwa 31,5 mmHg. Der PₑₜCO₂-Wert allein hat aber in der Praxis oftmals keine genügend hohe Sensitivität und Spezifität, um definitive diagnostische Aussagen zu machen. Wie oben erläutert, fließen in diesen Grenzwert auch noch weitere Parameter ein. Dieser Grenzwert wird daher in der beschriebenen Anwendung angewendet, aber in den Score fließen im Anschluss in oben beschriebener Weise noch nicht dynamische Parameter ein.

Ist der gemessene PₑₜCO₂ größer oder gleich G1, so ist der Scorewert 1 und eine PH als unwahrscheinlich erachtet.

Ist der gemessene PₑₜCO₂ kleiner oder gleich G1 so wird die Diagnose PH als zunächst wahrscheinliche erachtet (Scorewert S gleich 0).

Anschließend erfolgt anhand weiterer Parameter welche aus den EKG-Messdaten bestimmt wurden(Sokolow-Lyon-Index, S-Zacke in V5 (SV5), R-Zacke in V2 (RV2) und Winkel der QRS-Achse) und vorzugsweise aus MRT-Bilddaten bestimmten Parametern eine weitere Klassifizierung unter Beachtung der bereits existenten Grenzwerte.

Ein besonders vorteilhafter Parameter P1 ist der Sokolow-Lyon-Index welcher sich aus den EGK-Messdaten ergibt.

Der Sokolow-Lyon-Index ist ein unspezifisches EKG-Zeichen für eine vorliegende Links- oder Rechtsherzhypertrophie. Zur Berechnung des Sokolow-Index werden die Brustwandableitungen nach Wilson verwendet. Ein in der Fachwelt derzeit anerkannter Grenzwert G2 für den Sokolow-Lyon Index liegt bei 0,5.

Ist der gemessene Sokolow-Lyon Index größer oder gleich G2, so ist der Scorewert gleich 1 und eine PH als unwahrscheinlich erachtet.

Dazu addiert man die zunächst höchste Amplitude (in mV) der S-Zacke in Ableitung V1 oder V2 und der R-Zacke in V5 oder V6. Überschreitet die Summe der beiden Amplituden 3,5 mV (35,0 mm), spricht dies für eine PH.

Die Berechnung des Sokolow-Index für die RVH erfolgt ebenfalls mit V1 oder V2 bzw. V5 oder V6, nur dass man die jeweils andere Zacke verwendet. Man addiert den Ausschlag der R-Zacke in V1 oder V2 mit dem Ausschlag der S-Zacke in V5 oder V6. Wenn die Summe beider Amplituden größer als 1,05 mV (10,5 mm) ist, spricht das für das wahrscheinliche Vorliegen einer PH.

Der Sokolow-Lyon-Index allein hat aber eine ungenügend hohe Sensitivität und Spezifität, um definitive diagnostische Aussagen zu machen. Deshalb wird er zur Bestimmung des Scorewertes S wenigstens mit den Messdaten für den endtidalen CO₂ Partialdruck (PₑₜCO₂) kombiniert. Als unspezifisches EKG-Zeichen kann der Sokolow-Lyon-Index jedoch zur Formulierung begründeter Verdachtsdiagnosen beitragen.

Falls der Sokolow-Lyon Index kleiner G₂ ist, so wird das Verfahren fortgesetzt und auf die S-Zacke in V5 geachtet und diese mit einem Grenzwert G3 verglichen. Ein gängiger Grenzwert G3 für die S-Zacke in V5 ist 0,575mV. Ist die S-Zacke in V5 größer oder gleich G3, so wird eine PH erneut als unwahrscheinlich erachtet und es gilt S gleich 1. Wenn dies nicht der Fall ist, wird das das Verfahren fortgesetzt und auf R-Zacke in V2 geachtet und diese mit einem Grenzwert G4 verglichen. Ein gängiger Grenzwert G4 die auf R-Zacke in V2 ist 0,125mV. Ist die R-Zacke in V2 kleiner G4, so ist S gleich 1. Wenn dies nicht der Fall ist, wird das Verfahren fortgesetzt und die QRS Achse beachtet und deren Winkel mit einem Grenzwert G5 verglichen. Ein gängiger Grenzwert G5 für den Winkel der QRS Achse liegt bei 65°. Ist der Winkel der QRS Achse kleiner G5 ist S gleich 1, falls der Winkel der QRS Achse kleiner oder gleich 65° ist, ist S gleich 0.

Die Grenzwerte G1, G2, G3 G4 und G5 für PₑₜCO₂, Sokolow-Lyon Index, SV5, RV2 und für den Winkel der QRS-Achse entsprechen den derzeitig gängigen Grenzwerten für Patenten ohne Vorerkrankungen und wurden aus Vergleichsstudien ermittelt.

Dieser Scorewert S für das Vorliegen einer Pulmonalen Hypertonie ist ein für die Diagnose relevantes Zwischenergebnis und muss von ärztlichem Fachpersonal für eine Diagnose im medizinischen Kontext noch interpretiert werden. Beispielsweise kann der Schwellwert für das Vorliegen einer Pulmonalen Hypertonie ein Scorewert S von 0,5 sein. Werte von 0,5 oder kleiner werden als Pulmonale Hypertonie identifiziert. Dieser Schwellenwert wurde von ärztlichem Fachpersonal auf Grund von Erfahrung beispielhaft ermittelt und ist nicht Teil des erfindungsgemäßen Verfahrens.

Bei der Auswertung der Messdaten ist entscheidend, wie groß die Wahrscheinlichkeit ist, dass eine Pulmonale Hypertonie vorliegt bzw. wie schwerwiegend diese ist. Die Interpretation des Scorewertes S zur Erstellung einer konkreten Diagnose ist ärztlichem Fachpersonal vorbehalten und nicht Teil des Verfahrens. Ein typischer Schwellwert für das Vorliegen einer Pulmonalen Hypertonie kann beispielweise 0,5 sein; das heißt Werte von 0,5 oder kleiner werden als hohe Wahrscheinlichkeit für das Vorliegen einer (relevanten) Pulmonalen Hypertonie identifiziert.

Die Kombination der Messdaten über den endtidalen CO₂ Partialdruckes mit Messdaten wenigstens eines weiteren Parameters P1 des Herz-Lungen-Systems über den gleichen Zeitraum t führt dabei zu einer wesentlich sichereren und zuverlässigeren Diagnostik einer Pulmonalen Hypertonie, die nicht-invasiv am Patienten durchführbar ist.

Das erfindungsgemäße Verfahren ermöglicht auch die Eingruppierung der Wahrscheinlichkeit der Pulmonalen Hypertonie aus dem endtidalen CO₂ Partialdruck der ausgeatmeten Atemluft.

Im Rahmen des erfindungsgemäßen Verfahrens wird auf Basis einer breit verfügbaren Diagnosemethode (Messung des endtidalen CO₂ Partialdruckes) das Ergebnis einer nur eingeschränkt verfügbaren Diagnosemethode (z. B. Rechtsherzkatheter) vorhergesagt und damit werden die diagnostischen Möglichkeiten der endtidalen CO₂-Messung erweitert.

### Ausführungsbeispiel

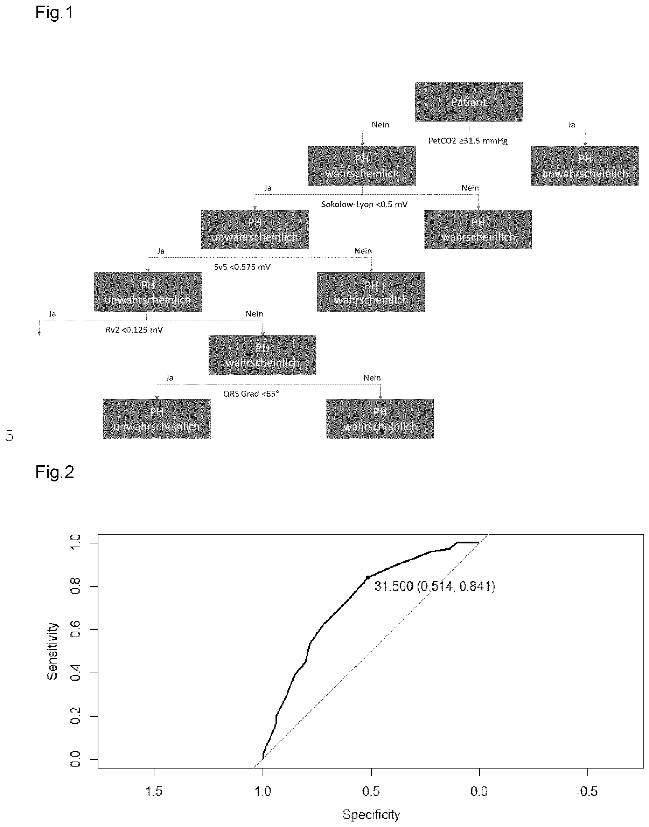

Als Beispiel in der Abbildung Fig.2 wird der Nutzen des endtidalen CO₂ Partialdruck für die Detektion einer präkapillären PH (PAH oder chronisch thrombembolische PH) gezeigt. Die Diagnose "PH" bzw. "Ausschluss PH" wurde dabei invasiv mittels Rechtsherzkatheter in Vergleichsuntersuchungen überprüft:
Der Wert des endtidalen CO₂ Partialdruck per se zeigt beim nach Youden bestimmten, optimalen Cut-Off von 31,5 mmHg eine Sensitivität von 84%. Die Youden Methode ist ein Index, welches als Maß für einen optimalen Grenzwert bei Receiver-Operating-Characeristic Curve Analysen verwendet wird. Jedoch ist die Spezifität stark eingeschränkt, sie beträgt nur 51%:
Die Hinzunahme von EKG-Messdaten wie dem Sokolow-Lyon Index sowie der Höhe der R-Zacke bzw. S-Zacke in verschiedenen Ableitungen erhöht sowohl die Sensitivität als auch Spezifität der Detektionsmethode (Sensitivität: 86%, Spezifität: 68%). Hier erfolgt wie oben bereits erwähnt die Klassifikation anhand des PₑₜCO₂. Anschließend wird bspw. bei Patienten mit hohem PetCO₂ ein positiver Sokolow-Lyon Index als Rechtsherzbelastungszeichen gewertet, so dass eine Umklassifizierung von "PH unwahrscheinlich" zu "PH wahrscheinlich" erfolgt. Das zeigt, dass es vorteilhaft ist, den Messwert PₑₜCO₂ mit weiteren Parametern zu kombinieren.

Bei dem gezeigten Ausführungsbeispiel handelt es sich lediglich um ein Beispiel für den Nutzen einer Kombination von PₑₜCO₂ mit einem weiteren Parameter P1 in einer vorläufigen Untersuchungskohorte und ist daher als beispielhaftes Prozedere zu verstehen.

In einem Beispielfall hat ein Patient X einen PetCO₂ von 30.7 mmHg. Dieser Wert istdamit kleiner der Grenzwer G1 von 31.5 mmHg). Eine PH ist daher zunächst wahrscheinlich. Zur weiteren Differenzierung wurde dann der Sokolow-Lyon-Index bestimmt. Dieser ist kleiner 0.5 ist. Daher ist PH weiterhin unwahrscheinlich. Mit dem nächsten Schritt wurde der, Sv5 Wert bestimmt. Dieser ist 0.3 Damit erscheint Pulmonale Hypertonie weiterhin unwahrscheinlich. Anschließend wurde der Rv2 bestimmt. Dieser liegt bei liegt bei 0.2, damit ist eine Pulmonale Hypertonie noch unwahrscheinlich. Abschließend wurde der Winkel der QRS Achse des Patienten bestimmt. Dieser liegt bei 61° und ist damit kleiner als der Grenzwert G5 von 65° und somit keine Indikation für PH und damit eine geringe Wahrscheinlichkeit für PH damit S gleich 1.

Die Vorhersage, dass bei diesem Patienten keine Pulmonale Hypertonie vorliegt, konnte durch eine komplementäre Diagnostik mittels Rechtsherzkatether bestätigt werden.

### Abbildungen:

Fig.1 zeigt den Algorithmus für die Bestimmung des Scorewertes. Die Grenzwerte in diesem Beispiel entsprechen den derzeitig gängigen Grenzwerten für Patienten ohne Vorerkrankungen.
Fig.2 zeigt die Selektivität und Sensitivität bei der Bestimmung einer Detektion einer PH.

## Patentansprüche

1. Verfahren zur Ermittlung eines Scorewertes (S) für die Wahrscheinlichkeit des Vorliegens einer Pulmonalen Hypertonie (PH) für das Vorliegens einer Pulmonalen Hypertonie einer Person (P) wenigstens aufweisend folgende Schritte:
I. Bereitstellen der CO₂-Messdaten über den endtidalen CO₂ Partialdruckes (PetCO₂) der Person (P) über einen Zeitraum (t)
II. Bereitstellen von EKG-Messdaten und Bestimmung wenigstens des Wertes des Sokolow-Lyon Index , des Wertes der S-Zacke in V5, des Wertes der R-Zacke in V4 und des Wertes des Winkels der QRS-Achse aus den EKG-Messdaten der Person (P) als dynamische-klinische Parameter (P1) über den gleichen Zeitraum (t) wie in Schritt I
III. Bestimmung eines Scorewertes (S) für die Wahrscheinlichkeit des Vorliegens einer Pulmonalen Hypertonie dieser Person aus den CO₂-Messdaten über den endtidalen CO₂ Partialdruckes (PetCO₂) aus Schritt I und wenigstens der EKG-Messdaten als dynamischer-klinischer Parametern (P1) aus Schritt II in folgenden Teilschritten, wobei S gleich 0 eine hohe Wahrscheinlichkeit einer Pulmonalen Hypertonie (PH) bedeutet und S gleich 1 eine hohe eine geringe Wahrscheinlichkeit einer Pulmonalen Hypertonie (PH) bedeutet
a. Vergleich des PetCO₂ aus Schritt I mit einem Grenzwert G1,
• bei PₑtCO₂ größer oder gleich G1 ist S gleich 1,
• bei PₑₜCO₂ kleiner G1, erfolgt eine Weiterführung des Verfahrens in Schritt IIIb,
b. Vergleich des Wertes Sokolow-Lyon Index aus Schritt II mit einem Grenzwert G2,
• bei Sokolow-Lyon Index größer oder gleich G2 ist S gleich 1,
• bei Sokolow-Lyon Index < G2 erfolgt eine Weiterführung des Verfahrens in Schritt IIIc,
c. Vergleich des Wertes der S-Zacke in V5 aus Schritt II mit einem Grenzwert G3,
• bei S-Zacke in V5 größer oder gleich G3 ist S gleich 0,
• bei S-Zacke in V5 <G3 erfolgt eine Weiterführung des Verfahrens in Schritt IIId,
d. Vergleich des Wertes der R-Zacke in V4 aus Schritt II mit einem Grenzwert G4,
• bei R-Zacke in V2 kleiner G4, ist S gleich 1,
• bei R-Zacke in V2 größer oder gleich G4 erfolgt eine Weiterführung des Verfahrens in Schritt IIIe,
e. Vergleich des Wertes des Winkels der QRS-Achse aus Schritt II mit einem Grenzwert G5,
• bei Winkel der QRS Achse kleiner G5 ist S gleich 1,
• bei Winkel der QRS Achse kleiner oder gleich G5 ist S gleich 0.
